# EUROPEAN PATENT APPLICATION

(11) **EP 4 063 005 A1**
(43) Date of publication of application: **28.09.2022**
(21) Application number: 19953327.4
(22) Date of filing: 22.11.2019
(51) Int. Cl.: B01D 67/00, C02F 1/26, C07C 209/86

(54) **SUPERHYDROPHOBIC MEMBRANE AND PREPARATION METHOD THEREFOR, AND METHOD FOR CONCENTRATING AND RECYCLING MDI WASTE BRINE**

(71) Applicant: Wanhua Chemical Group Co., Ltd., Yantai, Shandong 264006 (CN); Wanhua Chemical (Ningbo) Co., Ltd., Ningbo, Zhejiang 315812 (CN)
(72) Inventor: LI, Yongfeng, Yantai, Shandong 264006 (CN); XING, Jinming, Yantai, Shandong 264006 (CN); WU, Xuefeng, Yantai, Shandong 264006 (CN); ZHANG, Hongke, Yantai, Shandong 264006 (CN); FAN, Zhenlong, Yantai, Shandong 264006 (CN); GAO, Xueshun, Yantai, Shandong 264006 (CN); ZENG, Fanxue, Yantai, Shandong 264006 (CN); CUI, Chengcheng, Yantai, Shandong 264006 (CN); ZHOU, Bo, Yantai, Shandong 264006 (CN); ZHAO, Yibing, Yantai, Shandong 264006 (CN); LI, Peng, Yantai, Shandong 264006 (CN); YANG, Qilin, Yantai, Shandong 264006 (CN)
(74) Representative: HGF
(86) International application number: PCT/CN2019/120339
(87) International publication number: WO 2021/097819

(57) **Abstract**

A superhydrophobic membrane and a preparation method therefor, and a method for concentrating and recycling MDI waste brine. The method for concentrating and recycling MDI waste brine comprises: 1) performing two-phase separation on MDI waste brine to produce an organic phase and a brine phase; 2) sequentially subjecting the brine phase to aniline extraction, reboil distillation, and TOC removal processes; and 3) washing the organic phase to obtain washing wastewater; enabling the washing wastewater to contact an extractant by means of the superhydrophobic membrane for membrane extraction; and transporting the washing wastewater having experienced the membrane extraction to a biochemical unit and then enabling same to enter a reclaimed water recycling system.

## Description

### TECHNICAL FIELD

The present disclosure belongs to the technical field of treatment of industrial waste brine and, in particular, relates to a superhydrophobic membrane suitable for extracting high-concentration organic amine wastewater, a preparation method thereof and a method for concentrating and recycling methylene diphenyl diisocyanate (MDI) waste brine.

### BACKGROUND

MDI is one of the major feedstocks in the polyurethane industry. A well-known method in the industry for preparing MDI is as follows: in the presence of an acidic catalyst, aniline and formaldehyde are reacted to prepare polymethylene polyphenyl polyamine (DAM), and DAM and phosgene are reacted to synthesize MDI. In a process of preparing DAM from aniline and formaldehyde, plenty of high-concentration brine is produced after the obtained reaction solution is neutralized by caustic soda. Moreover, a small amount of salt is entrained in an organic phase obtained after the neutralization. The salt needs to be removed through further washing to ensure the stable operation of a subsequent process and stable and qualified DAM.

In the conventional process, neutralized brine after the neutralization with caustic soda and washing wastewater obtained after the washing have substantially the same organic composition. Therefore, the two are generally mixed together in whole or in part for extraction, distillation and total organic carbon (TOC) removal and then recycled for use as raw materials of chloralkali or discharged into sea.

For example, the patent document CN200880107822.2 provides a treatment process in which neutralized brine and washing wastewater are combined for extraction and steam stripping. The patent document CN200980118981.7 provides a process in which toluene is used as a solvent for extraction and steam stripping evaporation. However, in the above processes of mixing the neutralized brine and the washing wastewater for the subsequent treatment, since a brine content in the washing wastewater is far lower than that in the neutralized brine, the brine obtained after the treatment has a low concentration of salt, which not only limits an amount of recycling of a downstream chloralkali apparatus and results in a large discharge amount into the sea but also needs further concentration of the brine to meet a requirement for the recycling, thereby resulting in an increase in energy consumption and cost.

Similarly, many well-known methods may be used for concentrating the brine, such as multieffect evaporation and mechanical vapor recompression (MVR). However, to date, no one has mentioned the following method: the neutralized brine is completely separated from the washing wastewater during the process of preparing DAM, and the two are treated separately to achieve complete resource utilization of wastewater in the production process of DAM

Therefore, it is urgent to develop such a simple and efficient process of concentrating and treating MDI waste brine with a low cost from a process source to achieve complete resource utilization of a waste salt and wastewater in the production process of MDI.

### SUMMARY

In view of the problems in an existing process of concentrating MDI waste brine, an object of the present disclosure is to provide a superhydrophobic membrane, a preparation method thereof and a method for concentrating and recycling MDI waste brine. Neutralized brine and washing wastewater in the MDI waste brine are treated separately, which can concentrate the brine and completely recycle the washing wastewater on the premise of low cost and low energy consumption. After the treatment performed by the method of the present disclosure, high-concentration brine having sodium chloride with a mass percentage content of 21-25% is obtained. Moreover, the superhydrophobic membrane of the present disclosure simplifies a process flow of the treatment, fundamentally avoiding steam consumption during a treatment process of the washing wastewater.

To achieve the preceding object, the present disclosure adopts technical solutions described below.

In an aspect, a preparation method of a superhydrophobic membrane for extracting high-concentration organic amine wastewater is provided. The preparation method includes the following steps:
i. preparing a bionic template: an epoxy resin adhesive is uniformly coated on a glass substrate, and the epoxy resin adhesive on the glass substrate is uniformly covered with micron-sized silicon carbide particles; and after curing and baking, silicon carbide particles unfixed on a surface of the epoxy resin adhesive are cooled and removed to form a bionic template similar to a lotus effect;
ii. preparing a fluorosilicone rubber membrane with rough surface: a liquid fluorosilicone rubber (FVMQ), a curing agent and a solvent are uniformly mixed to form a casting solution, and then the obtained casting solution is uniformly coated on a surface of the bionic template; and the casting solution is cured into a membrane at room temperature, heated and dried, and stripped to obtain the fluorosilicone rubber membrane (FVMQ membrane) with the rough surface;
iii. performing plasma surface treatment: the fluorosilicone rubber membrane is subjected to the plasma surface treatment to obtain a fluorosilicone rubber membrane having an increased membrane porosity; and
iv. the obtained fluorosilicone rubber membrane having the increased membrane porosity is immersed in a surface modification liquid for surface modification; and then subjected to thermal curing to obtain the superhydrophobic membrane.

According to the preparation method provided in the present disclosure, preferably, in step i, each of the micron-sized silicon carbide particles has a particle size of 0.5-15 µm (for example, 1 µm, 3 µm,5 µm, 10 µm, 12 µm), more preferably 2-8 µm.

In some examples, the epoxy resin adhesive is a medium-temperature curing adhesive and includes an epoxy resin and a curing agent a.

In some preferred embodiments, the epoxy resin is selected from one or more of a bisphenol A-type epoxy resin, a bisphenol F-type epoxy resin, a bisphenol S-type epoxy resin and a bisphenol P-type epoxy resin; and the curing agent a is selected from one or more of dicyandiamide, diethylimidazole and methyl tetracyanophthalic anhydride.

In step i, for example, the prepared glass substrate with the epoxy resin adhesive covered with the micron-sized silicon carbide particles may be placed in an oven, where silicon carbide should be used in an amount such that the epoxy resin adhesive is at least completely covered, that is, silicon carbide should be covered at a thickness of at least 4 mm above a height of the surface of the epoxy resin adhesive. In some examples, a condition of the curing includes a curing temperature of 90-110 °C (for example, 100 °C, 105 °C) and a curing time of 50-80 mins (for example, 60 mins, 70 mins). In some examples, a condition of the baking includes a baking temperature of 150-170 °C (for example, 155 °C, 160 °C, 165 °C) and a baking time of 8-15 mins (for example, 10 mins, 12 mins).

In step i, the surface of the bionic template may be cooled to 100 °C or lower after the baking. The surface is then uniformly blown using an apparatus (for example, a blower) for 10-20 mins to remove the silicon carbide particles unfixed on the surface of the template.

The bionic template prepared with the micron-sized silicon carbide particles in step i has very many tiny papillae on the surface, which have an average diameter similar to that of papillae on a surface of a lotus leaf so that the surface of the bionic template has relatively good roughness.

According to the preparation method provided in the present disclosure, in step ii, in some examples, the curing agent is selected from at least one of vinyltriamine, dipropylenetriamine and triethylenetetramine. In some examples, the solvent is acetone and/or ethanol. For example, the curing agent, the solvent and the liquid fluorosilicone rubber may be stirred for 0.5-1 h at a rotational speed of 300 r/min to form the casting solution.

In some preferred embodiments, a mass ratio of the liquid fluorosilicone rubber to the curing agent to the solvent is 100:(5-8):(15-30), for example, the mass ratio is 100:6:20, 100:6:25, 100:6.5:28.

In step ii, after the casting solution is uniformly coated on the surface of the bionic template (that is, coated on a surface where the silicon carbide particles are fixed), for example, the solvent therein may be allowed to volatilize naturally at room temperature (for example, 25 °C) so that casting solution is cured into the membrane. In some examples, the casting solution is cured into the membrane at room temperature for 10-18 h (for example, 12 h, 14 h, 16 h).

In step ii, for example, the membrane cured into at room temperature may be moved to an oven for heating and drying. In some examples, a condition of the heating and drying includes a temperature of 70-95 °C (for example, 75 °C, 80 °C, 85 °C, 90 °C) and a time of 4-6 h (for example, 5 h). After dried in the oven, the membrane on the glass substrate is stripped to obtain the FVMQ membrane with the rough surface.

Since shapes of silicon carbide particles are random and gaps between particles have different shapes and sizes, when the casting solution with a certain viscosity is coated on the surface of the bionic template covered with the silicon carbide particles, the casting solution fills the gaps between the silicon carbide particles, and after the drying and the stripping, a thin membrane having a rough microstructure opposite to the morphology of the template is obtained. The preparation of this rough surface makes superhydrophobic performance of the membrane possible.

In addition, an FVMQ material is selected and used as it has good resistance to an acid, an alkali, an organic solvent and a high temperature of lower than 200 °C and is suitable for an operation situation where amine-containing organic wastewater is extracted using the acid.

According to the preparation method provided in the present disclosure, in step iii, for example, the FVMQ membrane obtained in step ii may be fixed on a carrier plate of a plasma surface treatment apparatus. The plasma surface treatment apparatus is switched on, an ammonia-containing gas (for example, ammonia, a mixed gas of ammonia and nitrogen or a mixed gas of ammonia and air) is introduced, and parameters are set to operate a plasma generator for surface treatment.

In some preferred embodiments, conditions of the plasma surface treatment include a radio frequency power of 10-150 W (for example, 30 W, 50 W, 80 W, 100 W, 120 W), a gas flowrate of 0-1000 cc/min (for example, 1 cc/min, 5 cc/min, 10 cc/min, 50 cc/min, 100 cc/min, 300 cc/min, 500 cc/min, 800 cc/min) and a vacuum degree of 60-600 Pa (for example, 80 Pa, 100 Pa, 150 Pa, 200 Pa, 300 Pa, 500 Pa), and the membrane is treated for 1-5 min (for example, 2 mins, 3 mins, 4 mins) each time and 2-5 times (for example, 3 times, 4 times) intermittently.

In this step, the surface of the obtained fluorosilicone rubber membrane is treated by plasma so that pores on the surface of the membrane are distributed more uniformly and the porosity is increased, which is conducive to improving a treatment capability and a treatment effect in a process of the membrane extraction.

According to the preparation method provided in the present disclosure, in step iv, in some examples, the surface modification liquid includes a substance with low surface energy, an alcohol solvent, a cross-linker and a catalyst. In a process of immersing the obtained fluorosilicone rubber membrane having the increased membrane porosity in the surface modification liquid, the surface modification liquid may be used in an amount such that the membrane immersed therein is completely immersed.

Preferably, the substance with low surface energy is selected from at least one of perfluorodecyltriethoxysilane, perfluorooctyltriethoxysilane and 3,3,3-trifluoropropyltrimethoxysilane.

Preferably, the alcohol solvent is isopropanol.

Preferably, the cross-linker is tetraethyl orthosilicate.

Preferably, the catalyst is aqueous ammonia.

More preferably, in the surface modification liquid, a molar ratio of the substance with low surface energy to isopropanol to tetraethyl orthosilicate to aqueous ammonia (based on an amount of a solute therein) is 5:(70-90):2:4, for example, the molar ratio is 5:75:2:4, 5:80:2:4 or 5:85:2:4.

In some examples, the surface modification is performed for 2-4 h, for example, 2.5 h, 3 h, 3.5 h.

After the surface modification is finished, the treated membrane may be subjected to solvent volatilization at room temperature for 6-8 h and to the thermal curing (for example, moved to an oven). In some examples, a condition of the thermal curing includes a temperature of 70-100 °C (for example, 80 °C, 90 °C) and a time of 8-15 h (for example, 10 h, 12 h, 14 h).

In another aspect, a superhydrophobic membrane prepared by the above preparation method is provided. The superhydrophobic membrane has a porous structure and a rough surface.

In some preferred embodiments, the superhydrophobic membrane has a thickness of 0.5-1.5 mm (for example, 0.6 mm, 1 mm, 1.2 mm), more preferably 0.6-0.8 mm.

In some preferred embodiments, the superhydrophobic membrane has a porosity of 10%-20% (for example, 12%, 14%, 16%, 18%).

In some preferred embodiments, the superhydrophobic membrane has a static water contact angle of 150°-160° (for example, 152°, 155°, 158°).

The superhydrophobic membrane of the present disclosure is a superhydrophobic fluorosilicone rubber membrane. The membrane may be used in an extraction membrane tube placed in a membrane extraction apparatus in a process of washing wastewater treatment. Compared with an ordinary separation membrane, the superhydrophobic membrane, as a porous superhydrophobic fluorosilicone rubber membrane, has the advantages of high selectivity to organic substances such as an amine, high permeability of an organic amine, resistance to an acid and swelling of organic substances and is particularly applicable to an operation situation of amine-containing organic wastewater in the productive process of MDI.

The superhydrophobic membrane of the present disclosure has very high selectivity to organic substances such as aniline, DAM and trace cyclohexylamine contained in washing wastewater and can effectively prevent inorganic ions and water molecules from passing the membrane so that it is feasible to treat the washing wastewater through membrane extraction.

In another aspect, a method for concentrating and recycling MDI waste brine is provided. The method includes the following steps:
(1) performing two-phase separation on MDI waste brine to produce an organic phase and a brine phase;
(2) sequentially subjecting the brine phase obtained in step (1) to treatment processes of aniline extraction, reboiling distillation and brine advanced treatment to remove TOC, and the treated brine is used as raw materials of a chloralkali apparatus; and
(3) washing the organic phase obtained in step (1) to obtain a washed organic phase and washing wastewater; enabling the washing wastewater to contact an extractant for membrane extraction; and transporting the washing wastewater subjected to the membrane extraction to a biochemical unit for treatment and then enabling same to enter a reclaimed water recycling system for recycling.

During a process of the membrane extraction, the washing wastewater to be treated flows through a superhydrophobic membrane prepared by the above preparation method or the above superhydrophobic membrane for the membrane extraction.

In the present disclosure, the MDI waste brine to be treated may be a product obtained after the neutralization of a mixed solution of a sodium hydroxide solution and a reaction solution containing diamine of diphenyl-methane series and DAM, where the reaction solution is prepared by reacting aniline with formaldehyde in the presence of a catalyst (for example, hydrochloric acid). The neutralization of the reaction solution containing diamine of diphenylmethane series and DAM is well-known to those skilled in the art and not repeated here.

The brine phase obtained after the two-phase separation in step (1) is a brine solution containing aniline and DAM, and the main composition of the brine phase generally includes NaCl with a mass fraction of 17-22%, NaOH with a mass fraction of 0.5-2%, and aniline and DAM with a total mass fraction of 0.2-3%.

According to the method provided in the present disclosure, the two-phase separation in step (1) is well-known to those skilled in the art and not repeated here.

According to the method provided in the present disclosure, the treatment processes of the aniline extraction, the reboiling distillation and the brine advanced treatment to remove TOC performed in sequence on the brine phase in step (2) are well-known to those skilled in the art and not repeated here. For example, the technology of hypo-sodium catalytic oxidation for TOC removal, which is disclosed in the patent document CN201610719105.1, may be used.

In some examples, the treated brine in step (2) includes NaCl having a content of 21-25wt%, TOC of ≤ 10 mg/L and total nitrogen (TN) of ≤ 3 mg/L. Compared with brine obtained through an existing treatment process, the treated brine includes NaCl whose content is increased by 6-8wt% since the MDI waste brine is effectively concentrated in this step. Therefore, in the case where a production capacity of the chloralkali apparatus is matched with that of an MDI apparatus, the MDI waste brine can be fully recycled for use as raw materials of chloralkali.

The washing wastewater to be treated in step (3) is washing wastewater separated from the organic phase and obtained after the organic phase is washed, and the washing wastewater to be treated includes aniline with a mass percentage content of 1-3%, DAM with a mass percentage content of less than 1%, NaCl with a mass percentage content of less than 1000 mg/L, and NaOH with a mass percentage content of less than 200 ppm.

The process of washing the organic phase in step (3) is well-known to those skilled in the art and not repeated here.

According to the method provided in the present disclosure, in some examples, the membrane extraction is performed in a membrane extraction apparatus. In step (3), the membrane extraction apparatus may select to use a conventional membrane extractor, for example, a shell-and-tube membrane extractor, which is special in that the superhydrophobic membrane in the present disclosure is used in an extraction membrane tube layer of the membrane extraction apparatus.

In some preferred embodiments, the superhydrophobic membrane is disposed in a tube layer of the membrane extraction apparatus, and the extractant is placed in a shell layer of the membrane extraction apparatus.

During the membrane extraction in step (3), the washing wastewater to be treated enters the tube layer of the membrane extraction apparatus, and the extractant is located in the shell layer of the membrane extraction apparatus. Substances such as aniline, diphenylmethane diamine and DAM contained in the washing wastewater to be treated can enter the extractant through the superhydrophobic membrane in the tube layer and react with the extractant into substances such as aniline hydrochloride, diamine hydrochloride and polyamine hydrochloride which cannot pass through the superhydrophobic membrane so that the amine substances such as aniline, diphenylmethane diamine and DAM which are present in the extractant in the form of amine molecules always have a concentration of zero. In this way, it is ensured that a concentration difference driving force always exists on both sides of the superhydrophobic membrane during the membrane extraction, thereby ensuring a mass transfer velocity during the membrane extraction. The obtained crude product containing aniline hydrochloride, diamine hydrochloride and polyamine hydrochloride after the extraction may be recycled to the MDI apparatus for use as part of raw materials for a condensation reaction of aniline and formaldehyde.

According to the method provided in the present disclosure, in some examples, the extractant is an aqueous hydrochloric acid solution having a mass concentration of 5%-40%, preferably 30%-37%.

In some examples, a ratio of feed volume flow rates of the extractant to the washing wastewater is 0.1-0.3 (for example, 0.12, 0.16, 0.18, 0.22, 0.25), preferably 0.15-0.20.

In some examples, a feed temperature of the extractant may be controlled at 20-35 °C.

In some examples, a temperature of the washing wastewater at an inlet of the tube layer of the membrane extraction apparatus is controlled at 40-60 °C, preferably at 45-50 °C.

In some examples, a mean residence time of the extractant and the washing wastewater in the membrane extraction apparatus is 40-80 mins, preferably 50-60 mins.

The biochemical treatment process performed in the biochemical unit and the reclaimed water recycling process performed in the reclaimed water recycling system in step (3) are well-known to those skilled in the art and not repeated here.

In the present disclosure, the brine phase obtained in step (1) is no longer mixed with any amount of washing wastewater obtained after the organic phase is washed for a subsequent treatment process; instead, the brine phase is treated separately according to step (2), and the washing wastewater obtained after the organic phase is washed is treated separately according to the treatment process in step (3).

After the membrane extraction, the crude product containing aniline hydrochloride, diamine hydrochloride and polyamine hydrochloride and the treated washing wastewater can be obtained. In step (3), the washing wastewater subjected to the membrane extraction includes DAM of ≤ 1 mg/L, aniline of ≤ 100 mg/L and a chemical oxygen demand (COD) of ≤ 500 mg/L. The washing wastewater subjected to the membrane extraction can meet a requirement of treatment in the biochemical unit. The crude product containing aniline hydrochloride, diamine hydrochloride and polyamine hydrochloride and obtained after the extraction can be directly recycled as a catalyst for the condensation reaction of aniline and formaldehyde.

The present disclosure develops a simple and efficient treatment method for concentrating waste brine in a production process of DAM with a low cost from a process source to achieve complete resource utilization. In this method, any amount of washing wastewater having a low concentration of salt and obtained after an organic phase is washed is no longer mixed with neutralized brine; instead, the neutralized brine (a brine phase) is separately subjected to solvent extraction, distillation and TOC removal to form high-concentration brine having sodium chloride with a mass fraction of 21-25%. Compared with an existing treatment process in which the washing wastewater and the neutralized brine are mixed, the method improves a concentration of the treated brine by 6-8%, which solves the following problem: in the case where a production capacity of chloralkali is matched with that of MDI, MDI waste brine cannot be fully recycled.

Meanwhile, the present disclosure develops a process for separately treating and recycling the washing wastewater. If a treatment method consistent with that of the neutralized brine is used, the process has the risks of a small two-phase density difference, too large a size of the plant and an unstable effect during aniline extraction; and if a solvent is replaced with another solvent such as toluene as mentioned in the patent document CN200980118981.7 for extraction, new impurities are introduced and the treatment process becomes complicated. In the present disclosure, a process of "membrane extraction" is combined with a process of "biochemical + reclaimed water recycling" so that an extractant is no longer in direct contact with the washing wastewater to be treated during the process of the membrane extraction, which simplifies a process flow and fundamentally avoids steam consumption during the treatment process of the washing wastewater.

Compared with an existing treatment process of mixing the neutralized brine and the washing wastewater during the treatment of the MDI waste brine, the overall method of the present disclosure has lower energy consumption and high separation efficiency while improving the concentration of the by-product brine of MDI, thereby achieving complete resource utilization of the neutralized brine and the washing wastewater in the MDI waste brine.

Compared with the existing art, technical solutions of the present disclosure have beneficial effects described below:
The porous superhydrophobic fluorosilicone rubber membrane of the present disclosure has uniformly distributed pores, no defect and a rough surface, where a fluorosilicone rubber layer is made at one time and firmly adhered to a modification layer with low surface energy. The membrane has high selectivity and high permeability to organic amine substances and is particularly applicable to the operation situation of the MDI waste brine.

In the present disclosure, the aqueous hydrochloric acid solution is preferably used as the extractant and enters the membrane extraction apparatus (where the superhydrophobic membrane prepared with a fluorosilicone rubber material is disposed in the tube layer of the apparatus) to extract organic amine substances in the washing wastewater so that the washing wastewater to be treated is not in direct contact with the extractant (the aqueous hydrochloric acid solution), avoiding emulsification and solvent separation in a conventional solvent extraction process. While the process is simplified, the wastewater treatment has the lower energy consumption, and the crude product containing aniline hydrochloride, diamine hydrochloride and polyamine hydrochloride after the extraction can be further recycled as the raw material for the condensation reaction of aniline and formaldehyde, which is more green and environmentally friendly than the conventional process. The washing wastewater in the production process of MDI can be treated by the method to the washing wastewater composed of COD of ≤ 500 mg/L, DAM of ≤ 1 mg/L and aniline of ≤ 100 mg/L, which meets the requirement of the subsequent biochemical treatment. After the biochemical treatment, the washing wastewater can be recycled in the reclaimed water recycling system.

In the present disclosure, the neutralized brine (the brine phase) in the MDI waste brine is no longer mixed with any amount of washing wastewater obtained after the organic phase is washed and neutralized for the subsequent treatment; instead, the brine phase and the washing wastewater are separately subjected to different treatment processes to obtain the high-concentration brine having sodium chloride with a mass percentage content of 21-25%, which concentrates the MDI waste brine and improves the content of NaCl in the treated brine by 6-8wt%. In the case where the production capacity of the chloralkali apparatus is matched with that of the MDI apparatus, the MDI waste brine can be fully recycled for use as the raw material of chloralkali.

Therefore, on the premise of energy conservation and cost saving, the present disclosure achieves the complete resource recycling of the MDI waste brine and the wastewater in the production process of MDI.

### DETAILED DESCRIPTION

To understand technical features and contents of the present disclosure in detail, preferred embodiments of the present disclosure are described in more detail below. Although the preferred embodiments of the present disclosure are described in examples, it is to be understood that the present disclosure may be implemented in various forms and is not limited to the embodiments set forth herein.

### <Sources of Raw Materials>

An epoxy resin adhesive is provided by *Guangzhou Jinshengji Chemical Co., Ltd.*

A liquid fluorosilicone rubber (FVMQ) with a viscosity of 60-90 Pa·s (25 °C) is provided by *Shanghai Shudi Fluorosilicon Materials Co., Ltd.*

Both triethylenetetramine (an analytical reagent), and vinyltriamine and dipropylenetriamine (an analytical reagent), are provided by *Chengdu Aikeda Chemical Reagent Co., Ltd.*

3,3,3-Trifluoropropyltrimethoxysilane with a purity of 98% is provided by *Hubei Jiangmin Taihua Chemical Co., Ltd.*

Perfluorodecyltriethoxysilane with a purity of 98% is provided by *Quzhou Dongming Chemical Co., Ltd.*

Perfluorooctyltriethoxysilane with a purity of 98% is provided by *Quzhou Dongming Chemical Co., Ltd.*

Isopropanol (an analytical reagent) is provided by *Ningbo Changyuan Instrument Co., Ltd.*

Tetraethyl orthosilicate (an analytical reagent) is provided by *Tianjin Fuchen Chemical Reagent Plant.*

An ordinary silicone rubber membrane material is a polydimethylsiloxane (PDMS) membrane provided by *Silex Ltd. of the United Kingdom.* The membrane is a hydrophobic dense membrane which consists of fumigated silicon dioxide of 30wt% and PDMS of 70wt% and has a porosity of 4.75%, a thickness of 1.0 mm and a static water contact angle of 101°.

MDI waste brine to be treated includes plenty of high-concentration brine and washing wastewater, where the high-concentration brine is produced after a reaction solution obtained in a reaction of aniline and formaldehyde for DAM preparation is neutralized by caustic soda, and the washing wastewater is produced after an organic phase is washed and neutralized.

### <Test Method>

A membrane extraction apparatus has a structure similar to that of a shell-and-tube heat exchanger, including a shell, a sealing head, a tube sheet and an extraction membrane tube. The extraction membrane tube is made of a fluorosilicone rubber superhydrophobic membrane prepared in the present disclosure, an inlet and an outlet for the washing wastewater are disposed at sealing heads at two ends, and the sealing heads at the two ends are connected to tube sheets fixed at two ends of the shell through flanges. An inlet and an outlet for an extractant (an aqueous hydrochloric acid solution) are disposed on the shell, multiple baffle support plates are disposed inside the shell, and the shell has an inner diameter of 2.5 m. The extraction membrane tube has a length of 6 m, an inner diameter of 15 mm and a thickness of 0.5-1.5 mm, the number of membrane tubes is 450, and a distance between the baffle support plates is 500 mm.

Test Methods for Performance Parameters of Membrane Obtained in Each Example:
(1) A porosity of the membrane is measured by using a full-automatic AutoPore mercury porosimeter developed by Micromeritics Instruments Corporation in the United States, where the pressure is set to 40 psi.
(2) A thickness of the membrane is analyzed by using a scanning electron microscope (SEM). The prepared fluorosilicone rubber superhydrophobic membrane is frozen in liquid nitrogen and cut quickly and longitudinally with a blade to prepare a sample. The thickness of the measured sample of the membrane is analyzed by using an S-4800 SEM developed by *Hitachi, Ltd. of Japan.*
(3) A static water contact angle is measured as follows: a fixed image is measured after 0.2 µL of water drops is dropped on an active surface of the prepared fluorosilicone rubber superhydrophobic membrane for 10s, at room temperature and under a humidity of 60%, and using a JC2000D1 contact angle meter developed by *Shanghai Zhongchen Digital Technic Apparatus Co., Ltd.*

Method for Measuring a Content of Each Component in a Brine Phase and Washing Wastewater in Each Example and Comparative Example:
A content of salt (such as sodium hydroxide, sodium chloride, etc.) is measured through potentiometric titration.
Contents of aniline and DAM are measured through liquid chromatography (LC).
A content of COD is measured through potassium dichromate oxidation.
Contents of TOC and TN are measured through combustion by using a TOC analyzer developed by Analytik Jena AG of Germany.

### Example 1

1. The superhydrophobic membrane used in the membrane extraction apparatus was prepared according to steps described below.
   i. A bionic template was prepared: (a) an epoxy resin adhesive was uniformly coated on a glass substrate, and the epoxy resin adhesive on the glass substrate was uniformly covered with micron-sized silicon carbide particles, where each of the silicon carbide particles has a particle size of 5 µm; (b) the glass substrate covered with the micron-sized silicon carbide particles was placed in an oven, cured at 100 °C for 80 mins, baked at 160 °C for 15 mins, taken out and cooled to below 100 °C; and (c) a surface of the epoxy resin adhesive was uniformly blown using a blower for 15 mins to remove silicon carbide particles unfixed on the surface of the epoxy resin adhesive to form a bionic template similar to a lotus effect.
   ii. A fluorosilicone rubber membrane with a rough surface was prepared: a liquid fluorosilicone rubber (FVMQ), a curing agent and a solvent were mixed at a mass ratio of 100:8:20 and stirred for 0.5 h at a rotational speed of 300 r/min to form a casting solution, where the curing agent was triethylenetetramine and the solvent was acetone, and then the casting solution was uniformly coated on a surface of the prepared bionic template where the silicon carbide particles were fixed; the solvent was volatilized naturally at room temperature for 15 h, the casting solution was cured into a membrane, the membrane was moved to the oven and dried at 80 °C for 6 h, and the membrane on the glass substrate was stripped to obtain the FVMQ membrane having the rough surface.
   iii. Plasma surface treatment was performed: the obtained FVMQ membrane was fixed on a carrier plate of a plasma surface treatment apparatus, the treatment apparatus was turned on, ammonia was introduced, parameter conditions were set as a radio frequency power of 120 W, a gas flowrate of 600 cc/min and a vacuum degree of 300 Pa, and a base membrane was treated for 3 min each time and 3 times intermittently, and then the fluorosilicone rubber membrane was sealed and stored to obtain a fluorosilicone rubber membrane with an increased membrane porosity, where the membrane porosity was increased from 6.23% to 19.58%.
   iv. 3,3,3-Trifluoropropyltrimethoxysilane, isopropanol, tetraethyl orthosilicate and aqueous ammonia were prepared into a surface modification liquid according to a molar ratio of 5:80:2:4; the FVMQ membrane having the increased membrane porosity and obtained in step iii was completely immersed in the surface modification solution and subjected to surface modification for 3 h; after the reaction, the solvent was volatilized naturally at room temperature for 8 h, and the membrane was moved to the oven for thermal curing at 90 °C for 15 h to obtain a porous fluorosilicone rubber superhydrophobic membrane I with a static water contact angle of 159° and a thickness of 0.8 mm.
2. The prepared fluorosilicone rubber superhydrophobic membrane I was disposed in a tube layer of the membrane extraction apparatus and used in a method for concentrating and recycling MDI waste brine.
3. The method for concentrating and recycling the MDI waste brine includes steps described below.
   (1) The MDI waste brine to be treated was introduced into a layer separator at 100-108 °C and stayed for 20 mins for two-phase separation to obtain an organic phase and a brine phase. Neutralized brine (that is, the obtained brine phase) includes: NaCl with the mass fraction of 18.5%, NaOH with the mass fraction of 0.9%, and aniline and DAM with the total mass fraction of 0.9% and wastewater has an amount of 80 m³/h.
   (2) The neutralized brine was separately subjected to treatment processes of aniline extraction, reboiling distillation and brine advanced treatment to remove TOC in sequence (during the treatment process of the aniline extraction, a feed mass ratio of aniline to the neutralized brine was 0.22:1, a feed temperature of aniline was 30 °C, and a feed temperature of the neutralized brine was 100 °C; the treatment process of the reboiling distillation was performed using a distillation column with a thermosyphon reboiler and not refluxed, steam (2 kg) was used for heating the reboiler, a feed mass ratio of steam to the neutralized brine was 0.14, and a feed temperature of the neutralized brine was 95 °C; and during the treatment process of the brine advanced treatment to remove TOC, the neutralized brine was cooled to 50 °C and fed at a pH adjusted to 12, mixed with an aqueous sodium hypochlorite solution (5%) at a mass ratio of 100:1 at 30 °C, and entered a fixed bed column reactor, and a catalyst was loaded in the column reactor in an amount such that a space velocity of the feeding was 5BV/h), where the treated brine has a flowrate of 65 m³/h. Compared with a treatment process where a brine phase is mixed with washing wastewater, the treatment increases a content of NaCl in the obtained brine by 6.7wt%. The treated brine includes: NaCl with a content of 22.5wt%, TOC with a content of 6.7 mg/L, and TN with a content of 1.2 mg/L. All the treated brine may be used as raw materials of a chloralkali apparatus.
   (3) The organic phase obtained in step (1), aniline water produced in a DAM refining unit and aniline water produced in the reboiling distillation process of the neutralized brine (wherein, the aniline water in the DAM refining unit was derived from the following process: the mixed material containing water (9%), aniline (21%) and DAM (70%) and having a temperature of 95 °C was heated to 105 °C and subjected to flash evaporation under an absolute pressure of 12 KPa, a liquid phase after the flash evaporation was heated to 200 °C and introduced into a stripping column, steam (8 kg) was introduced into the bottom of the stripping column under an absolute pressure of 9 KPa according to a feed mass ratio 0.08:1 of the steam to the mixed material having a temperature of 95 °C for stripping, and a gas phase of the aniline water produced through the flash evaporation and at the top of the stripping column was cooled to 20 °C and allowed to stand in the layer separator for 25 mins to separate an aniline phase and a water phase 1, where the water phase 1 was the aniline water produced in the DAM refining unit; the reboiling distillation process of the neutralized brine was as follows: aniline water produced at the top of the column was cooled to 20 °C and allowed to stand in the layer separator for 25 mins to separate an aniline phase and a water phase 2, where the water phase 2 was the aniline water produced in the reboiling distillation of the neutralized brine; the water phase 1 and the water phase 2 were combined as aniline water for washing the organic phase obtained in step (1)) were mixed in a stirring tank at 90-98 °C for washing, entered the layer separator and stayed for 25 mins to obtain the washed organic phase and washing wastewater. The washing wastewater includes: aniline with a mass fraction of 2.5%, DAM with a mass fraction of 0.7%, NaCl with a content of 500 mg/L, and NaOH with a content of 100 ppm, and the wastewater has an amount of 35 m³/h.

The washing wastewater to be treated and the aqueous hydrochloric acid solution as the extractant were delivered to a membrane tube layer and a shell layer of the membrane extraction apparatus respectively for membrane extraction, wherein the aqueous hydrochloric acid solution had a mass concentration of 30%, a ratio of volume flow rates of the aqueous hydrochloric acid solution to the washing wastewater was 0.2, the temperature of hydrochloric acid at an inlet of the shell layer was 30 °C, the temperature of the washing wastewater at an inlet of the membrane tube was 45 °C, and the average residence time for the extraction was 50 mins. The washing wastewater subjected to the membrane extraction includes COD with a content of 321 mg/L, DAM with a content of 0.5 mg/L, and aniline with a content of 63 mg/L. The washing wastewater was delivered to a biochemical unit for further treatment and then to a reclaimed water recycling unit for recycling. The obtained crude product containing aniline hydrochloride, diamine hydrochloride and polyamine hydrochloride after the extraction may be recycled to an MDI apparatus for use as part of raw materials for a condensation reaction of aniline and formaldehyde.

### Example 2

1. The superhydrophobic membrane used in the membrane extraction apparatus was prepared according to steps described below:
   i. A bionic template was prepared: (a) an epoxy resin adhesive was uniformly coated on a glass substrate, and the epoxy resin adhesive on the glass substrate was uniformly covered with micron-sized silicon carbide particles, where each of the silicon carbide particles had a particle size of 3 µm; (b) the glass substrate covered with the micron-sized silicon carbide particles was placed in an oven, cured at 100 °C for 60 mins, baked at 160 °C for 15 mins, taken out and cooled to below 100 °C; and (c) a surface of the epoxy resin adhesive was uniformly blown using a blower for 15 mins to remove silicon carbide particles unfixed on the surface of the epoxy resin adhesive to form a bionic template similar to a lotus effect.
   ii. A fluorosilicone rubber membrane with a rough surface was prepared: a liquid fluorosilicone rubber (FVMQ), a curing agent and a solvent were mixed at a mass ratio of 100:5:20 and stirred for 0.5 h at a rotational speed of 300 r/min to form a casting solution, where the curing agent was vinyltriamine and the solvent was acetone, and then the casting solution was uniformly coated on a surface of the prepared bionic template where the silicon carbide particles were fixed; the solvent was naturally volatilized at room temperature for 18 h, the casting solution was cured into a membrane, the membrane was moved to the oven and dried at 80 °C for 6 h, and the membrane on the glass substrate was stripped to obtain the FVMQ membrane with the rough surface.
   iii. Plasma surface treatment was performed: the obtained FVMQ membrane was fixed on a carrier plate of a plasma surface treatment apparatus, the treatment apparatus was turned on, ammonia and nitrogen were introduced into the plasma surface treatment apparatus according to a volume ratio of 1:1, parameter conditions were set as a radio frequency power of 100 W, a gas flowrate of 500 cc/min and a vacuum degree of 400 Pa, and a base membrane was treated for 2 mins each time and 4 times intermittently, and then the FVMQ membrane was sealed and stored to obtain a fluorosilicone rubber membrane with an increased membrane porosity, where the membrane porosity was increased from 6.47% to 16.58%.
   iv. Perfluorodecyltriethoxysilane, isopropanol, tetraethyl orthosilicate and aqueous ammonia were prepared into a surface modification liquid according to a molar ratio of 5:75:2:4; the FVMQ membrane having the increased membrane porosity and obtained in step iii was completely immersed in the surface modification solution and subjected to surface modification for 4 h; after the reaction, the solvent was volatilized naturally at room temperature for 8 h, and the membrane was moved to the oven for thermal curing at 90 °C for 15 h to obtain a porous fluorosilicone rubber superhydrophobic membrane II having a static water contact angle of 155° and a thickness of 0.6 mm.
2. The prepared fluorosilicone rubber superhydrophobic membrane II was disposed in a tube layer of the membrane extraction apparatus and used in a method for concentrating and recycling MDI waste brine. The membrane extraction apparatus has the same structure and the same size as that in Example 1.
3. The method for concentrating and recycling the MDI waste brine includes the steps described below.
   (1) The MDI waste brine to be treated was introduced into a layer separator at 100-108 °C and stayed for 20 mins for two-phase separation to obtain an organic phase and a brine phase. Neutralized brine (that is, the obtained brine phase) includes NaCl with a mass fraction of 20.5%, NaOH with a mass fraction of 1.2%, and aniline and DAM with a total mass fraction of 1.5%, and wastewater has an amount of 80 m³/h.
   (2) The neutralized brine was separately subjected to treatment processes of aniline extraction (see Example 1 for a specific treatment process), reboiling distillation (see Example 1 for a specific treatment process) and brine advanced treatment to remove TOC (see Example 1 for a specific treatment process) in sequence, where the treated brine has a flowrate of 65 m³/h. Compared with a treatment process where a brine phase is mixed with washing wastewater, the treatment increases a content of NaCl in the treated brine by 7.6wt%. The treated brine includes: NaCl with a content of 24wt%, TOC with a content of 5.5 mg/L, and TN with a content of 0.8 mg/L. All the treated brine may be used as raw materials of a chloralkali apparatus.
   (3) The organic phase obtained in step (1), and aniline water produced in a DAM refining unit and aniline water produced in the reboiling distillation process of the neutralized brine (see Example 1 for a preparation process of the aniline water) were mixed in a stirring tank at 90-98 °C for washing, and then entered the layer separator for 25 mins to obtain the washed organic phase and washing wastewater. The washing wastewater includes: aniline with the mass fraction of 2.2%, DAM with the mass fraction of 0.4%, NaCl with a content of 300 mg/L, and NaOH with a content of 60 ppm, and the wastewater has an amount of 35 m³/h.

The washing wastewater to be treated and the aqueous hydrochloric acid solution as the extractant were delivered to a membrane tube layer and a shell layer of the membrane extraction apparatus respectively for membrane extraction, wherein the aqueous hydrochloric acid solution had a mass concentration of 34%, a ratio of volume flow rates of the aqueous hydrochloric acid solution to the washing wastewater was 0.15, the temperature of hydrochloric acid at an inlet of the shell layer was 35 °C, the temperature of the washing wastewater at an inlet of the membrane tube was 55 °C, and the average residence time for the extraction was 60 mins. The washing wastewater subjected to the membrane extraction includes: COD with a content of 270 mg/L, DAM with a content of 0.3 mg/L, and aniline with a content of 42 mg/L. The washing wastewater was delivered to a biochemical unit for further treatment and then to a reclaimed water recycling unit for recycling. The obtained crude product containing aniline hydrochloride, diamine hydrochloride and polyamine hydrochloride after the extraction may be recycled to an MDI apparatus for use as part of raw materials for a condensation reaction of aniline and formaldehyde.

### Example 3

1. The superhydrophobic membrane used in the membrane extraction apparatus was prepared with reference to the method described in Example 1. The differences were described as below.
   i. A bionic template was prepared: each silicon carbide particle has a particle size of 8 µm,and a glass substrate covered with silicon carbide particles was cured at 100 °C for 70 mins.
   ii. A fluorosilicone rubber membrane with a rough surface was prepared: a liquid fluorosilicone rubber (FVMQ), a curing agent and a solvent were mixed at a mass ratio of 100:7:30, where the curing agent was dipropylenetriamine and the solvent was ethanol.
   iii. Plasma surface treatment was performed: ammonia and air were introduced into a plasma surface treatment apparatus according to a volume ratio of 1:1, and parameter conditions were set as a radio frequency power of 80 W, a gas flowrate 700 cc/min and a vacuum degree of 200 Pa, and a base membrane was treated for 4 mins each time and twice intermittently; finally, a fluorosilicone rubber membrane having an increased membrane porosity was obtained, where the membrane porosity was increased from 6.47% to 11.59%.
   (4) Perfluorooctyltriethoxysilane, isopropanol, tetraethyl orthosilicate and aqueous ammonia were prepared into a surface modification liquid according to a molar ratio of 5:90:2:4, the fluorosilicone rubber membrane having the increased membrane porosity and obtained in step iii was completely immersed in the surface modification solution and subjected to surface modification for 2 h to obtain a porous fluorosilicone rubber superhydrophobic membrane III having a static water contact angle of 153° and a thickness of 1.2 mm.
2. The prepared fluorosilicone rubber superhydrophobic membrane III was disposed in a tube layer of the membrane extraction apparatus and used in a method for concentrating and recycling MDI waste brine. The membrane extraction apparatus has the same structure and the same size as that in Example 1.
3. The method for concentrating and recycling the MDI waste brine includes the steps described as below.
   (1) The MDI waste brine to be treated was introduced into a layer separator at 100-108 °C and stayed for 20 mins for two-phase separation to obtain an organic phase and a brine phase. Neutralized brine (that is, the obtained brine phase) includes: NaCl with a mass fraction of 19%, NaOH with a mass fraction of 0.6%, and aniline and DAM with a total mass fraction of 1.8%, and wastewater has an amount of 80 m³/h.
   (2) The neutralized brine was separately subjected to treatment processes of aniline extraction (see Example 1 for a specific treatment process), reboiling distillation (see Example 1 for a specific treatment process) and brine advanced treatment to remove TOC (see Example 1 for a specific treatment process) in sequence, where the treated brine has a flowrate of 65 m³/h. Compared with a treatment process where a brine phase is mixed with washing wastewater, the treatment increases a content of NaCl in the obtained brine by 6.9wt%. The treated brine includes: NaCl with a content of 23wt%, TOC with a content of 6.1 mg/L, and TN with a content of 0.9 mg/L. All the treated brine may be used as raw materials of a chloralkali apparatus.
   (3) The organic phase obtained in step (1), and aniline water produced in a DAM refining unit and aniline water produced in the reboiling distillation process of the neutralized brine (see Example 1 for a preparation process of the aniline water) were mixed in a stirring tank at 90-98 °C for washing, and then entered the layer separator for 25 mins to obtain the washed organic phase and washing wastewater. The washing wastewater includes: aniline with a mass fraction of 2.8%, DAM with a mass fraction of 0.6%, NaCl with a content of 700 mg/L, and NaOH with a content of 80 ppm, and the wastewater has an amount of 35 m³/h.

The washing wastewater to be treated and the aqueous hydrochloric acid solution as the extractant were delivered to a membrane tube layer and a shell layer of the membrane extraction apparatus respectively for membrane extraction, wherein the aqueous hydrochloric acid solution had a mass concentration of 25%, a ratio of volume flow rates of the aqueous hydrochloric acid solution to the washing wastewater was 0.2, the temperature of hydrochloric acid at an inlet of the shell layer was 30 °C, the temperature of the washing wastewater at an inlet of the membrane tube was 45 °C, and the average residence time for the extraction was 40 mins. The washing wastewater subjected to the membrane extraction includes: COD with a content of 453 mg/L, DAM with a content of 0.8 mg/L, and aniline with a content of 96 mg/L. The washing wastewater was delivered to a biochemical unit for further treatment and then to a reclaimed water recycling unit for recycling. The obtained crude product containing aniline hydrochloride, diamine hydrochloride and polyamine hydrochloride after the extraction may be recycled to an MDI apparatus for use as part of raw materials for a condensation reaction of aniline and formaldehyde.

### Example 4

1. The superhydrophobic membrane used in the membrane extraction apparatus was prepared with reference to the method described in Example 2. The differences were described as below:
   i. A bionic template was prepared: each silicon carbide particle has a particle size of 12 µm, and a glass substrate covered with silicon carbide particles was cured at 100 °C for 50 mins.
   ii. A fluorosilicone rubber membrane with a rough surface was prepared: a liquid fluorosilicone rubber (FVMQ), a curing agent and a solvent were mixed at a mass ratio of 100:6:25, where the curing agent was vinyltriamine and the solvent was ethanol.
   iii. Plasma surface treatment was performed: in a plasma surface treatment apparatus, parameter conditions were set as a radio frequency power of 50 W, a gas flowrate of 200 cc/min and a vacuum degree of 500 Pa, and a base membrane was treated for 5 mins each time and twice intermittently; finally, an fluorosilicone rubber membrane having an increased membrane porosity was obtained, where the membrane porosity was increased from 6.47% to 10.59%.
   iv. Perfluorodecyltriethoxysilane, isopropanol, tetraethyl orthosilicate and aqueous ammonia were prepared into a surface modification liquid according to a molar ratio of 5:70:2:4, the FVMQ membrane having the increased membrane porosity and obtained in step iii was completely immersed in the surface modification solution; after the reaction, the solvent was volatilized naturally at room temperature, and the membrane was moved to the oven for thermal curing at 100 °C for 10 h to obtain a porous fluorosilicone rubber superhydrophobic membrane IV with a static water contact angle of 150° and a thickness of 1.0 mm.
2. The prepared fluorosilicone rubber superhydrophobic membrane IV was disposed in a tube layer of the membrane extraction apparatus and used in a method for concentrating and recycling MDI waste brine. The membrane extraction apparatus has the same structure and the same size as that in Example 1.
3. The method for concentrating and recycling the MDI waste brine includes the steps described as below:
   (1) The MDI waste brine to be treated was introduced into a layer separator at 100-108 °C and stayed for 20 mins for two-phase separation to obtain an organic phase and a brine phase. Neutralized brine (that is, the obtained brine phase) includes: NaCl with a mass fraction of 17.5%, NaOH with a mass fraction of 1.8%, and aniline and DAM with a total mass fraction of 1.8%, and wastewater has an amount of 80 m³/h.
   (2) The neutralized brine was separately subjected to treatment processes of aniline extraction (see Example 1 for a specific treatment process), reboiling distillation (see Example 1 for a specific treatment process) and brine advanced treatment to remove TOC (see Example 1 for a specific treatment process) in sequence, where the treated brine has a flowrate of 65 m³/h. Compared with a treatment process where a brine phase is mixed with washing wastewater, the treatment increases a content of NaCl in the obtained brine by 7.3wt%. The treated brine includes: NaCl with a content of 23wt%, TOC with a content of 6.1 mg/L, and TN with a content of 0.9 mg/L. All the treated brine may be used as raw materials of a chloralkali apparatus.
   (3) The organic phase obtained in step (1), aniline water produced in a DAM refining unit and aniline water produced in the reboiling distillation process of the neutralized brine (see Example 1 for a preparation process of the aniline water) were mixed in a stirring tank at 90-98 °C for washing, entered the layer separator and stayed for 25 mins to obtain the washed organic phase and washing wastewater. The washing wastewater includes: aniline with a mass fraction of 1.9%, DAM with a mass fraction of 0.3%, NaCl with a content of 300 mg/L, and NaOH with a content of 40 ppm, and the wastewater has an amount of 35 m³/h.

The washing wastewater to be treated and the aqueous hydrochloric acid solution as the extractant were delivered to a membrane tube layer and a shell layer of the membrane extraction apparatus respectively for membrane extraction, where the aqueous hydrochloric acid solution had a mass concentration of 25%, a ratio of volume flow rates of the aqueous hydrochloric acid solution to the washing wastewater was 0.2, the temperature of hydrochloric acid at an inlet of the shell layer was 30 °C, the temperature of the washing wastewater at an inlet of the membrane tube was 45 °C, and the average residence time for the extraction was 40 mins. The washing wastewater subjected to the membrane extraction includes: COD with a content of 380 mg/L, DAM with a content of 0.7 mg/L, and aniline with a content of 82 mg/L. The washing wastewater was delivered to a biochemical unit for further treatment and then to a reclaimed water recycling unit for recycling. The obtained crude product containing aniline hydrochloride, diamine hydrochloride and polyamine hydrochloride after the extraction may be recycled to an MDI apparatus for use as part of raw materials for a condensation reaction of aniline and formaldehyde.

### Comparative Example 1

1. The membrane extraction apparatus used in a method of concentrating and recycling MDI waste brine has the same structure and the same size as that in Example 1, except that an ordinary silicone rubber membrane material (a PDMS membrane provided by *Silex Ltd. of the United Kingdom)* was used in a membrane tube layer of the membrane extraction apparatus.
2. The method for concentrating and recycling the MDI waste brine includes the steps described as below:
   (1) The MDI waste brine to be treated was introduced into a layer separator at 100-108 °C and stayed for 20 mins for two-phase separation to obtain an organic phase and a brine phase. Neutralized brine (that is, the obtained brine phase) includes: NaCl with a mass fraction of 20%, NaOH with a mass fraction of 1%, and aniline and DAM with a total mass fraction of 1.2%, and wastewater has an amount of 80 m³/h.
   (2) The organic phase obtained in step (1), aniline water produced in a DAM refining unit and aniline water produced in a reboiling distillation process of the neutralized brine (see Example 1 for a preparation process of the aniline water) were mixed in a stirring tank at 90-98 °C for washing, entered the layer separator and stayed for 25 mins to obtain the washed organic phase and washing wastewater. The washing wastewater includes: aniline with the mass fraction of 2.0%, DAM with the mass fraction of 0.5%, NaCl with a content of 400 mg/L, and NaOH with a content of 100 ppm, and the wastewater has an amount of 35 m³/h.
   (3) All the neutralized brine (that is, the brine phase) obtained in step (1) was mixed with the obtained washing wastewater having an amount of 20 m³/h in step (2), and the mixture was subjected to treatment processes of aniline extraction (see Example 1 for a specific treatment process), reboiling distillation (see Example 1 for a specific treatment process) and brine advanced treatment to remove TOC (see Example 1 for a specific treatment process) in sequence, where the treated brine has an amount of 80 m³/h. In the treated brine, NaCl has a content of 18.2wt%, TOC has a content of 7.1 mg/L, and TN has a content of 0.9 mg/L, and the brine with an amount of about 10 m³/h cannot be recycled.
   (4) Only the washing wastewater with an amount of 15 m³/h and the aqueous hydrochloric acid solution as the extractant were delivered to the membrane tube layer and a shell layer of the membrane extraction apparatus respectively for membrane extraction, where the aqueous hydrochloric acid solution had a mass concentration of 30%, a ratio of volume flow rates of the aqueous hydrochloric acid solution to the washing wastewater was 0.2, the temperature of hydrochloric acid at an inlet of the shell layer was 30 °C, the temperature of the washing wastewater at an inlet of the membrane tube was 45 °C, and the average residence time for the extraction was 50 mins. The washing wastewater subjected to the membrane extraction includes: COD with a content of 3240 mg/L, DAM with a content of 63 mg/L, and aniline with a content of 750 mg/L, which far exceeds a wastewater-receiving index of a biochemical system and cannot enter the biochemical system for further treatment.

### Comparative Example 2

1. The superhydrophobic membrane used in the membrane extraction apparatus was prepared with reference to the method described in Example 1, and the differences were that no plasma surface treatment in step iii was performed so that an FVMQ membrane V was obtained, where the FVMQ membrane V has a thickness of 0.8 mm, a porosity of 5.8% and a static water contact angle of 151°.
2. The prepared superhydrophobic fluorosilicone rubber membrane V was disposed in a tube layer of the membrane extraction apparatus and applied to a method for concentrating and recycling MDI waste brine. The membrane extraction apparatus has the same structure and the same size as that in Example 1.
3. The method for concentrating and recycling the MDI waste brine includes steps described below.
   (1) The MDI waste brine to be treated was introduced into a layer separator at 100-108 °C and stayed for 20 mins for two-phase separation to obtain an organic phase and a brine phase. Neutralized brine (that is, the obtained brine phase) includes: NaCl with a mass fraction of 20%, NaOH with a mass fraction of 1%, and aniline and DAM with a total mass fraction of 1.2%, and wastewater has an amount of 80 m³/h.
   (2) The organic phase obtained in step (1), aniline water produced in a DAM refining unit and aniline water produced in a reboiling distillation process of the neutralized brine (see Example 1 for a preparation process of the aniline water) were mixed in a stirring tank at 90-98 °C for washing, entered the layer separator and stayed for 25 mins to obtain the washed organic phase and washing wastewater. The washing wastewater includes: aniline with a mass fraction of 2.0%, DAM with a mass fraction of 0.5%, NaCl with a content of 400 mg/L, and NaOH with a content of 100 ppm, and the wastewater has an amount of 35 m³/h.
   (3) All the neutralized brine (that is, the brine phase) obtained in step (1) was mixed with the obtained washing wastewater having an amount of 30 m³/h in step (2), and the mixture was subjected to treatment processes of aniline extraction (see Example 1 for a specific treatment process), reboiling distillation (see Example 1 for a specific treatment process) and brine advanced treatment to remove TOC (see Example 1 for a specific treatment process) in sequence, where the treated brine has an amount of 88 m³/h. In the treated brine, NaCl has a content of 16.8wt%, TOC has a content of 6.4 mg/L, and TN has a content of 1.1 mg/L, and the brine with an amount of about 18 m³/h cannot be recycled.
   (4) Only the washing wastewater having an amount of 5 m³/h and the aqueous hydrochloric acid solution as the extractant were delivered to a membrane tube layer and a shell layer of the membrane extraction apparatus respectively for membrane extraction, where the aqueous hydrochloric acid solution had a mass concentration of 34%, a ratio of volume flow rates of the aqueous hydrochloric acid solution to the washing wastewater was 0.2, the temperature of hydrochloric acid at an inlet of the shell layer was 30 °C, the temperature of the washing wastewater at an inlet of the membrane tube was 45 °C, and the average residence time for the membrane extraction was 60 mins. The washing wastewater subjected to the membrane extraction includes: COD with a content of 1850 mg/L, DAM with a content of 27 mg/L, and aniline with a content of 320 mg/L, which far exceeds a wastewater-receiving index of a biochemical system and cannot enter the biochemical system for further treatment.

The following can be seen from the comparison of the results obtained in examples and comparative examples:
1. When the brine phase and the washing wastewater are subjected to different treatment processes respectively, high-concentration brine having sodium chloride with a mass percentage content of 21-25% can be obtained, the MDI waste brine is concentrated, and the content of NaCl in the treated brine is increased by 6-8wt%. In the case where a production capacity of the chloralkali apparatus is matched with that of the MDI apparatus, the MDI waste brine can be fully recycled for use as raw materials of chloralkali. In Comparative Examples 1 and 2, when the neutralized brine (the brine phase) in the MDI waste brine is mixed with any amount of washing wastewater obtained after the organic phase is washed and neutralized for a subsequent treatment, the MDI waste brine has a poor concentration effect and cannot be fully recycled for use as the raw material of chloralkali.
2. The fluorosilicone rubber superhydrophobic membrane prepared in the present disclosure is made of an FVMQ layer at one time and firmly adhered to a modification layer with low surface energy. The superhydrophobic membrane has higher permeability and higher selectivity for the extraction of organic amine substances in the MDI waste brine. Therefore, the washing wastewater can meet the wastewater-receiving index of a downstream biochemical system simply after one simple extraction. The treated washing wastewater has low contents of COD, DAM and aniline and is particularly applicable to an operation situation where MDI washing wastewater is extracted by hydrochloric acid. Moreover, the crude product containing aniline hydrochloride, diamine hydrochloride and polyamine hydrochloride after the extraction may be further recycled for use as the raw material for the condensation reaction, which is more green and environmentally friendly than the conventional process.

In addition, the treatment process is simple, efficient and stable, reducing the energy consumption.

The separate treatment and recycling of the neutralized brine and the washing wastewater in the MDI waste brine are achieved by using the fluorosilicone rubber superhydrophobic membrane obtained in the present disclosure. With the treatment capabilities (for example, the neutralized brine has an amount of 80 m³/h, and the washing wastewater has an amount of 35 m³/h) in the preceding examples and comparative examples, the steam consumption and resource recycling situations in the examples and comparative examples are shown in Table 1.

**Table 1 Steam consumption and resource recycling situation by using the treatment methods in the Examples and Comparative Examples**

| | Steam Consumption (t/h) | Loss of Brine Due to Drainage (m³/h) |
|---|---|---|
| Example 1 | 12 | 0 |
| Example 2 | 12 | 0 |
| Example 3 | 12 | 0 |
| Example 4 | 12 | 0 |
| Comparative Example 1 | 19 | 10 |
| Comparative Example 2 | 22 | 18 |

Various examples of the present disclosure have been described above. The above description is illustrative and not exhaustive, and the present disclosure is not limited to the disclosed various examples. Without departing from the scope and spirit of the described various examples, various modifications and variations are apparent to those of ordinary skill in the art.

## Claims

1. A preparation method of a superhydrophobic membrane for extracting high-concentration organic amine wastewater, comprising the following steps:
i. preparing a bionic template: an epoxy resin adhesive is uniformly coated on a glass substrate, and the epoxy resin adhesive on the glass substrate is uniformly covered with micron-sized silicon carbide particles; and after curing and baking, silicon carbide particles unfixed on a surface of the epoxy resin adhesive are cooled and removed to form a bionic template similar to a lotus effect;
ii. preparing a fluorosilicone rubber membrane with a rough surface: a liquid fluorosilicone rubber, a curing agent and a solvent are uniformly mixed to form a casting solution, and then the obtained casting solution is uniformly coated on a surface of the bionic template; and the casting solution is cured into a membrane at room temperature, heated and dried, and stripped to obtain the fluorosilicone rubber membrane having the rough surface;
iii. performing plasma surface treatment: the fluorosilicone rubber membrane is subjected to the plasma surface treatment to obtain a fluorosilicone rubber membrane with an increased membrane porosity; and
iv. the obtained fluorosilicone rubber membrane with the increased membrane porosity is immersed in a surface modification liquid for surface modification; and then subjected to thermal curing to obtain the superhydrophobic membrane.

2. The preparation method according to claim 1, wherein in step i:
the micron-sized silicon carbide particle has a particle size of 0.5-15 µm, preferably 2-8 µm;
the epoxy resin adhesive is a medium-temperature curing adhesive and comprises an epoxy resin and a curing agent a;
preferably, the epoxy resin is selected from one or more of a bisphenol A-type epoxy resin, a bisphenol F-type epoxy resin, a bisphenol S-type epoxy resin and a bisphenol P-type epoxy resin; and the curing agent a is selected from one or more of dicyandiamide, diethylimidazole and methyl tetracyanophthalic anhydride;
preferably, a condition of the curing comprises a curing temperature of 90-110 °C and a curing time of 50-80 mins; and a condition of the baking comprises a baking temperature of 150-170 °C and a baking time of 8-15 mins.

3. The preparation method according to claim 1 or 2, wherein in step ii:
the curing agent is selected from at least one of vinyltriamine, dipropylenetriamine and triethylenetetramine; and/or
the solvent is acetone and/or ethanol;
preferably, a mass ratio of the liquid fluorosilicone rubber to the curing agent to the solvent is 100:(5-8):(15-30);
preferably, the casting solution is cured into the membrane at room temperature for 10-18 h; and
preferably, a condition of the heating and drying comprises a temperature of 70-95 °C and a time of 4-6 h.

4. The preparation method according to any one of claims 1 to 3, wherein in step iii, conditions of the plasma surface treatment comprise a radio frequency power of 10-150 W, a gas flowrate of 0-1000 cc/min and a vacuum degree of 60-600 Pa, and the membrane is treated for 1-5 min each time and 2-5 times intermittently.

5. The preparation method according to any one of claims 1 to 4, wherein in step iv:
the surface modification liquid comprises a substance with low surface energy, an alcohol solvent, a cross-linker and a catalyst;
preferably, the substance with low surface energy is selected from at least one of perfluorodecyltriethoxysilane, perfluorooctyltriethoxysilane and 3,3,3-trifluoropropyltrimethoxysilane; the alcohol solvent is isopropanol; the cross-linker is tetraethyl orthosilicate; and the catalyst is aqueous ammonia;
more preferably, in the surface modification liquid, a molar ratio of the substance with low surface energy to isopropanol to tetraethyl orthosilicate to aqueous ammonia is 5:(70-90):2:4;
preferably, the surface modification is performed for 2-4 h;
preferably, a condition of the thermal curing comprises a temperature of 70-100 °C and a time of 8-15 h.

6. A superhydrophobic membrane prepared by the preparation method according to any one of claims 1 to 5, wherein the superhydrophobic membrane has a porous structure and a rough surface;
preferably, the superhydrophobic membrane has a thickness of 0.5-1.5 mm, more preferably 0.6-0.8 mm;
preferably, the superhydrophobic membrane has a porosity of 10%-20%; and
preferably, the superhydrophobic membrane has a static water contact angle of 150°-160°.

7. A method for concentrating and recycling methylene diphenyl diisocyanate, MDI, waste brine, comprising the following steps:
(1) performing two-phase separation on MDI waste brine to produce an organic phase and a brine phase;
(2) sequentially subjecting the brine phase obtained in step (1) is subjected to treatment processes of aniline extraction, reboiling distillation and brine advanced treatment to remove TOC, and the treated brine is used as raw materials of a chloralkali apparatus; and
(3) washing the organic phase obtained in step (1) to obtain a washed organic phase and washing wastewater; enabling the washing wastewater to contact an extractant for membrane extraction; and transporting the washing wastewater subjected to the membrane extraction to a biochemical unit for treatment and then enabling same to enter a reclaimed water recycling system for recycling;
wherein during a process of the membrane extraction, the washing wastewater to be treated flows through a superhydrophobic membrane prepared by the preparation method according to any one of claims 1 to 5 or the superhydrophobic membrane according to claim 6 for the membrane extraction.

8. The method according to claim 7, wherein the membrane extraction is performed in a membrane extraction apparatus; and
preferably, the superhydrophobic membrane is disposed in a tube layer of the membrane extraction apparatus, and the extractant is placed in a shell layer of the membrane extraction apparatus.

9. The method according to claim 7 or 8, wherein in step (3), the extractant is an aqueous hydrochloric acid solution having a mass concentration of 5%-40%, preferably 30%-37%.

10. The method according to claim 7 or 8, wherein the treated brine in step (2) comprises NaCl having a content of 21-25wt%, TOC of ≤ 10 mg/L and total nitrogen, TN, of ≤ 3 mg/L; and
in step (3), the washing wastewater subjected to the membrane extraction comprises polymethylene polyphenyl polyamine, DAM, of ≤ 1 mg/L, aniline of ≤ 100 mg/L and a chemical oxygen demand, COD, of ≤ 500 mg/L.
